# EUROPÄISCHE PATENTSCHRIFT

(11) **EP 2 008 085 B1**
(45) Veröffentlichungstag und Bekanntmachung des Hinweises auf die Patenterteilung: **19.10.2011**
(21) Anmeldenummer: 07701932.1
(22) Anmeldetag: 13.03.2007
(51) Int. Cl.: G01N 27/22, G01N 33/36, B65H 63/06

(54) **VERFAHREN UND VORRICHTUNG ZUR UNTERSUCHUNG VON BEWEGTEM, FESTEM, LÄNGLICHEM PRÜFGUT**
METHOD AND DEVICE FOR THE ANALYSIS OF MOVED, SOLID, ELONGATE TEST MATERIAL
PROCÉDÉ ET DISPOSITIF D'ANALYSE DE PRODUIT À VÉRIFIER ALLONGÉ, SOLIDE ET MOBILE

(30) Priorität: 07.04.2006 CH 5752006
(43) Veröffentlichungstag der Anmeldung: 31.12.2008
(73) Patentinhaber: Uster Technologies AG, 8610 Uster (CH)
(72) Erfinder: OTT, Philipp, CH-8496 Steg (CH)
(86) Internationale Anmeldenummer: PCT/CH2007/000137
(87) Internationale Veröffentlichungsnummer: WO 2007/115416

(56) Entgegenhaltungen:
- EP-A- 0 924 513
- DE-A1- 10 346 599
- US-A1- 2002 033 704

## Beschreibung

### FACHGEBIET

Die vorliegende Erfindung liegt auf dem Gebiet der Prüfung mit elektrischen Mitteln von festen, länglichen, vorzugsweise textilen Gebilden wie Kardenband, Vorgarn, Garn oder Gewebe. Sie betrifft ein Verfahren und eine Vorrichtung zur Untersuchung von bewegtem, festem, länglichem Prüfgut, gemäss den Oberbegriffen der unabhängigen Patentansprüche. Sie eignet sich insbesondere zur Erkennung fester Fremdstoffe wie Polypropylen im Prüfgut, zur Ermittlung einer Masse pro Längeneinheit des Prüfguts und/oder zur Ermittlung einer Feuchte des Prüfguts.

### STAND DER TECHNIK

In der Textilindustrie besteht Bedarf nach einer zuverlässigen Untersuchung von länglichen textilen Gebilden wie Garn. Insbesondere interessiert die Garnmasse pro Längeneinheit und die Erkennung von Fremdstoffen wie Polypropylen im Garn. Zu diesen Zwecken kommen häufig optische Sensoren zum Einsatz. Diese haben jedoch den Nachteil, dass sie Fremdstoffe nicht erkennen, welche für das verwendete Licht durchsichtig sind, dieselbe spektrale Reflektivität ("Farbe") wie das Prüfgut haben oder im Innern des Prüfguts versteckt und von aussen unsichtbar sind. Die Unzulänglichkeiten optischer Messprinzipien können durch den Einsatz von elektrischen, insbesondere kapazitiven Sensoren umgangen werden.

Um mehr Information über die Beschaffenheit des Garns und mögliche darin vorhandene Fremdstoffe zu erhalten, wurde schon vorgeschlagen, das Garn mit verschiedenen Sensoren abzutasten und die verschiedenen Sensorsignale zwecks Auswertung miteinander zu verknüpfen. So werden z. B. gemäss der WO-01/92875 A1 zwei Sensoren entlang des Garnpfades hintereinander angeordnet. Einer der Sensoren kann optisch, der andere optisch oder kapazitiv messen. Die serielle Anordnung zweier Sensoren hat den Nachteil, dass die Sensorsignale, die von einer bestimmten Garnstelle herrühren, nicht gleichzeitig, sondern nacheinander aufgenommen werden. Vor der Verknüpfung der Signale müssen die beiden Signale synchronisiert werden, d. h. ihre Zeitdifferenz muss eliminiert werden. Dazu wird die Garngeschwindigkeit benötigt, die jedoch variieren kann und nicht genau bekannt ist. In den Schriften EP-0'401'600 A2 und EP-0'884'409 A1 wird vorgeschlagen, mit einem optischen und einem kapazitiven Sensor gleichzeitig eine einzige Garnstelle abzutasten. Eine dazu erforderliche Verschachtelung zweier verschiedener Sensoren bringt jedoch konstruktive Schwierigkeiten mit sich: Die Messelektroden des kapazitiven Sensors müssen für das Licht des optischen Sensors durchlässig sein.

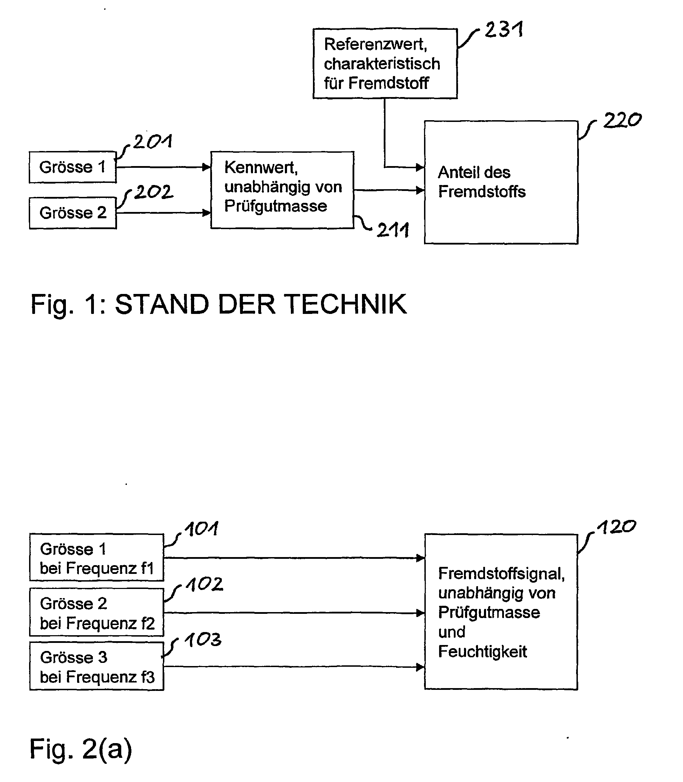

Aus der Es-0'924'513 A1 sind ein Verfahren und eine Vorrichtung zur kapazitiven Ermittlung von Anteilen fester Stoffe in textilem Prüfgut, z. B. Garn, bekannt. Dabei wird das Garn durch einen Plattenkondensator hindurch bewegt und einem elektrischen Wechselfeld ausgesetzt. Durch Messung der relativen Dielektrizitätszahl oder des Leistungsfaktors am Plattenkondensator werden dielektrische Eigenschaften des Garns ermittelt. Die Auswertung der dielektrischen Eigenschaften gemäss der EP-0'924'513 A1 ist in **Figur 1** dargestellt. Aus den dielektrischen Eigenschaften werden zwei elektrische Grössen 201, 202 ermittelt und zu einem Kennwert 211 kombiniert, welcher von der Garnmasse unabhängig ist. Der Kennwert 211 wird mit einem vorausgehend ermittelten Referenzwert 231 für einen bekannten festen Fremdstoff verglichen und daraus der Anteil 220 des festen Fremdstoffs bestimmt. Es werden zwei Möglichkeiten für die Ermittlung der zwei elektrischen Grössen 201, 202 angegeben. Eine erste Möglichkeit besteht in der Messung der Kapazitäten am Messkondensator bei zwei verschiedenen Frequenzen. Eine zweite Möglichkeit besteht darin, den Leistungsfaktor, d. h. die Phasenverschiebung zwischen Spannung und Strom, am Messkondensator bei einer einzigen Frequenz zu messen. Das Messprinzip gemäss EP-0'924'513 A1 vermag wohl den Anteil 220 eines bekannten festen Fremdstoffs im Garn zu ermitteln, kann jedoch nicht zwischen festen Stoffen und Feuchtigkeitsschwankungen des Garns unterscheiden. Es setzt voraus, dass die Feuchtigkeit der Garnfasern vor der Messung bekannt ist und während der Messung konstant gehalten wird. Diese Voraussetzung kann in der Praxis nicht immer erfüllt werden. Selbst in klimatisierten Räumen sind lokale Inhomogenitäten, Änderungen bzw. Schwankungen der Feuchtigkeit in den Garnfasern zu beobachten, geschweige denn in unklimatisierter Umgebung. Solche lokale Feuchtigkeitsschwankungen im Garn werden dann für Fremdstoffe gehalten, was zu Fehlansprechungen und bei Garnreinigern zu unnötigen Garnschnitten führt.

### DARSTELLUNG DER ERFINDUNG

Das bevorzugte Einsatzgebiet der vorliegenden Erfindung ist die Garnreinigung während des Spinn- oder Spulprozesses (online). Dabei soll in erster Linie eine optimale Garnreinigung gewährleistet werden, indem Fremdstoffe als solche erkannt und zuverlässig herausgeschnitten werden, während Feuchtigkeitsschwankungen keinen Schnitt auslösen. Eine quantitative Bestimmung des Anteils eines Fremdstoffs interessiert erst an zweiter Stelle. Es ist deshalb eine Aufgabe der vorliegenden Erfindung, das Verfahren und die Vorrichtung, wie sie aus der EP-0'924'513 A1 bekannt sind, derart weiter zu entwickeln, dass die Messung weitgehend unabhängig von lokalen Feuchtigkeitsschwankungen im Prüfgut wird. Insbesondere sollen feste Fremdstoffe wie Polypropylen zuverlässig erkannt und von lokalen Feuchtigkeitsschwankungen unterschieden werden.

Eine weitere Anwendung der vorliegenden Erfindung ist die Laboruntersuchung (offline) von länglichen textilen Gebilden. Hier interessieren insbesondre die Masse und die Feuchtigkeit des Garns, welche präzis und voneinander entkoppelt gemessen werden sollen. In der Praxis werden nämlich textile Laboruntersuchungen nicht immer in klimatisierten Räumen durchgeführt. Die Luftfeuchtigkeit und die Garnfeuchtigkeit entsprechen nicht immer den erwünschten genormten Standardbedingungen. Unter solchen Umständen kann es wichtig sein, die Garnfeuchtigkeit zu ermitteln, um die Resultate mit anderen Messungen oder internationalen Standards vergleichen zu können.

Diese und andere Aufgaben werden durch das Verfahren und die Vorrichtung gelöst, wie sie in den unabhängigen Patentansprüchen definiert sind. Vorteilhafte Ausführungsformen sind in den abhängigen Patentansprüchen angegeben.

Die Erfindung beruht auf der Erkenntnis, dass ein Ausgangssignal eines kapazitiven Garnsensors hauptsächlich durch die drei unbekannten Grössen Garnmasse, Garnfeuchtigkeit und Fremdstoffe beeinflusst wird. Um drei Unbekannte zu bestimmen, braucht es drei Gleichungen oder Messungen. Eine Kombination von nur zwei elektrischen Messgrössen, wie sie von der EP-0'924'513 A1 gelehrt wird, ist hierzu ungenügend. Gemäss der Erfindung wird mindestens eine weitere, dritte Grösse aus den dielektrischen Eigenschaften des Garns ermittelt. Aus drei Messgrössen lassen sich prinzipiell die drei Unbekannten Garnmasse, Garnfeuchtigkeit und Fremdstoffe bestimmen. Wenn eine dieser drei Unbekannten in der betreffenden Anwendung nicht interessiert, kann eine andere Unbekannte ermittelt werden, eine der zwei verbleibenden Unbekannten kann absolut statt nur relativ ermittelt werden, oder die verbleibenden Unbekannten können aus dem überbestimmten System durch Ausgleichsrechnung mit grösserer Genauigkeit ermittelt werden.

Im erfindungsgemässen Verfahren zur Untersuchung von bewegtem, festem, länglichem Prüfgut wird ein elektrisches Wechselfeld erzeugt, und das Prüfgut wird dem Wechselfeld ausgesetzt. Das Wechselfeld ist eine Überlagerung mindestens einer ersten Wechselfeldkomponente mit einer ersten Frequenz und einer zweiten Wechselfeldkomponente mit einer von der ersten Frequenz verschiedenen zweiten Frequenz. Es werden Eigenschaften des mit dem Prüfgut wechselwirkenden Wechselfeldes ermittelt, indem mindestens drei vom Wechselfeld beeinflusste elektrische Grössen bei jeweils einer Frequenz aufgenommen werden. Dabei werden eine der Grössen bei der ersten Frequenz und eine andere der Grössen bei der zweiten Frequenz aufgenommen. Die mindestens drei Grössen werden miteinander verknüpft. Bevorzugte Frequenzwerte sind ca. 10 MHz für die erste Frequenz und ca. 200 kHz für die zweite Frequenz.

Die erfindungsgemässe Vorrichtung zur Untersuchung von bewegtem, festem, länglichem Prüfgut beinhaltet einen Messkondensator mit einer Durchgangsöffnung für das Prüfgut. Ein Spannungsgenerator dient zum Anlegen einer Wechselspannung an den Messkondensator zwecks Beaufschlagung der Durchgangsöffnung mit einem elektrischen Wechselfeld. Der Spannungsgenerator ist geeignet, die Durchgangsöffnung mit einem Wechselfeld zu beaufschlagen, das eine Überlagerung mindestens einer ersten Wechselfeldkomponente mit einer ersten Frequenz und einer zweiten Wechselfeldkomponente mit einer von der ersten Frequenz verschiedenen zweiten Frequenz. Die Vorrichtung beinhaltet ferner eine mit dem Messkondensator elektrisch verbundene Auswerteschaltung zur gegenseitigen Verknüpfung von vom Wechselfeld beeinflussten elektrischen Grössen. Die Auswerteschaltung ist geeignet, mindestens drei vom Wechselfeld beeinflusste, bei jeweils einer Frequenz aufgenommene elektrische Grössen miteinander zu verknüpfen, wovon eine der Grössen bei der ersten Frequenz und eine andere der Grössen bei der zweiten Frequenz aufgenommen ist.

Bevorzugte Verwendungen der Erfindung sind die Erkennung fester Fremdstoffe wie Polypropylen im Prüfgut, die Ermittlung einer Masse pro Längeneinheit des Prüfguts und/oder die Ermittlung einer Feuchte des Prüfguts.

### AUFZÄHLUNG DER ZEICHNUNGEN

Nachfolgend wird die Erfindung und, zum Vergleich, der Stand der Technik anhand der Zeichnungen detailliert erläutert. Die Erläuterung erfolgt anhand von Beispielen auf dem Gebiet der Garnreinigung. Wie oben erwähnt, ist jedoch die Erfindung nicht auf diese Anwendung beschränkt. Die nachfolgende Diskussion ist daher keinesfalls als Einschränkung zu verstehen.
- Figur 1: veranschaulicht die Signalauswertung im Verfahren nach EP-0'924'513 A1.
- Figur 2: veranschaulicht auf analoge Weise wie Fig. 1 die Signalauswertung bei vier Ausführungsformen des erfindungsgemässen Verfahrens.
- Figur 3: zeigt in Diagrammen, in denen Messsignale gegenüber einer Position auf dem Garn aufgetragen sind, Signalverläufe im erfindungsgemässen Verfahren: (a) für einen zu erkennenden festen Fremdstoff und (b) für eine lokale Feuchtstelle.
- Figur 4: zeigt elektrische Schaltschemen von zwei Ausführungsformen der erfindungsgemässen Vorrichtung.

### AUSFÜHRUNG DER ERFINDUNG

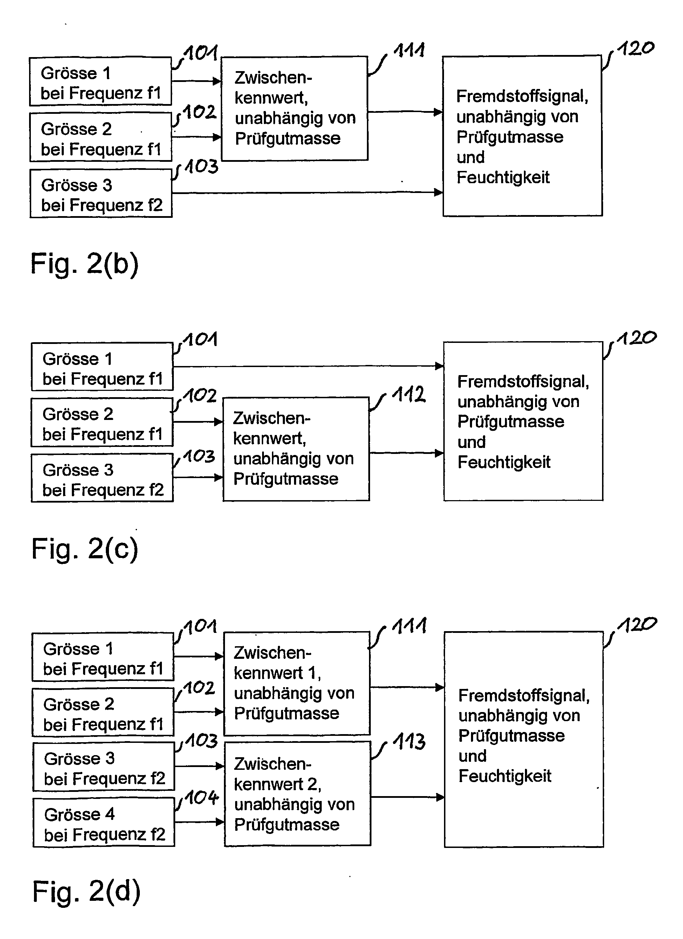

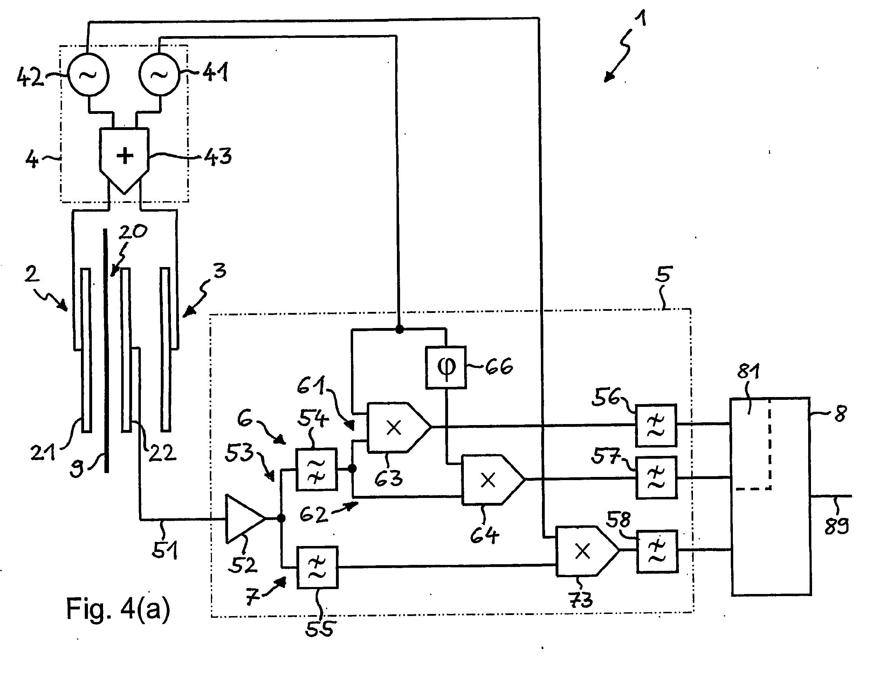

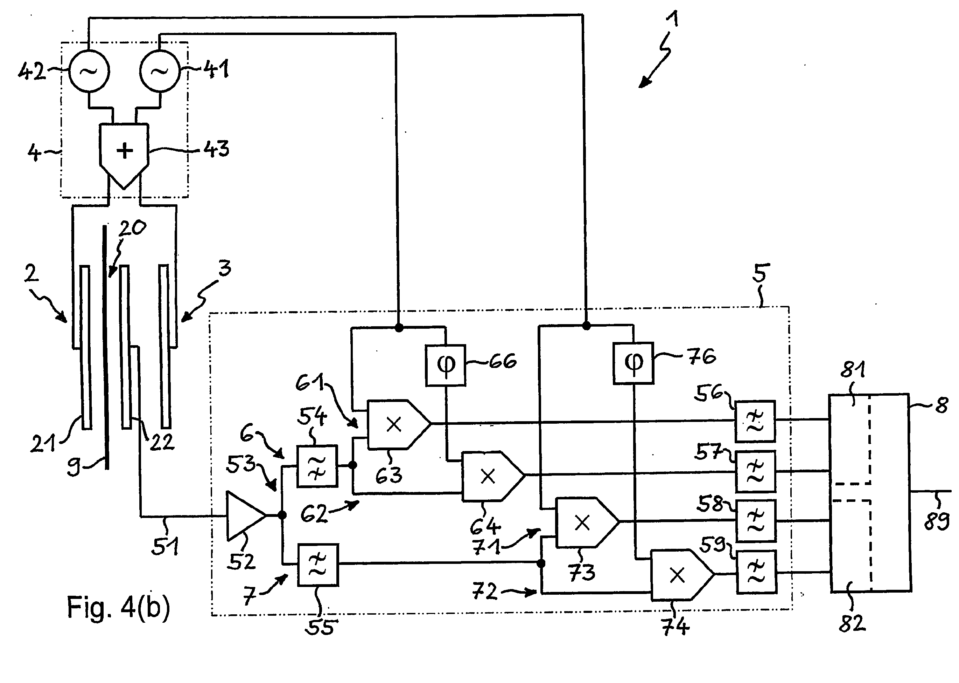

Im erfindungsgemässen Verfahren wird ein elektrisches Wechselfeld erzeugt, das mindestens zwei unterschiedliche Frequenzkomponenten beinhaltet. Dem Wechselfeld wird das Prüfgut ausgesetzt. **Figur 2** veranschaulicht die Signalauswertung beim erfindungsgemässen Verfahren in vier Varianten. Allen Varianten ist gemeinsam, dass mindestens drei vom Wechselfeld beeinflusste elektrische Grössen 101-104 bei jeweils einer Frequenz f1-f4 aufgenommen werden. Diese elektrischen Grössen 101-104 können z. B. an einem Messkondensator 2 (siehe Fig. 4) gemessene Ströme oder Spannungen sein. Die Frequenzen fl-f4, bei denen die elektrischen Grössen 101-104 aufgenommen werden, sind solche Frequenzen, die auch im Wechselfeld vorkommen. Sie werden vorzugsweise einerseits aus dem Bereich zwischen 1 MHz und 100 MHz, vorzugsweise zwischen 5 MHz und 50 MHz und bspw. ungefähr gleich 10 MHz, andererseits aus dem Bereich zwischen 10 kHz und 1000 kHz, vorzugsweise zwischen 50 kHz und 500 kHz und bspw. ungefähr gleich 200 kHz gewählt.

In der Ausführungsform von **Figur 2(a)** werden drei Grössen 101-103 bei jeweils einer Frequenz f1-f3 aufgenommen. Mindestens zwei der drei Frequenzen f1, f2, f3 sind ungleich, d. h. es gilt: ¬(f1 = f2 = f3); es können aber wohl zwei der drei Frequenzen gleich sein, z. B. f1 = f3 ≠ f2. Bevorzugte Frequenzwerte sind f1 = f3 = 10 MHz, f2 = 200 kHz. Die drei Grössen 101-103 werden miteinander verknüpft. Die Verknüpfung kann z. B. die Bildung einer geeigneten Linearkombination der drei Grössen 101-103 beinhalten. Durch die Verknüpfung entsteht ein Fremdstoffsignal 120, das von der Masse der Prüfguts und der Feuchtigkeit im Prüfgut im Wesentlichen unabhängig ist.

In der Ausführungsform von **Figur 2(b)** werden eine erste Grösse 101 und eine zweite Grösse 102 bei ein und derselben Frequenz f1, z. B. f1 = 10 MHz, aufgenommen, während eine dritte Grösse 103 bei einer anderen Frequenz f2 ≠ f1, z. B. f2 = 200 kHz, aufgenommen wird. Aus der ersten Grösse 101 und der zweiten Grösse 102 wird zunächst ein Zwischenkennwert 111 gebildet, der von der Masse des Prüfguts im Wesentlichen unabhängig ist. Der Zwischenkennwert 111 kann z. B. eine Phasenverschiebung, ein Leistungsfaktor oder ein Verlustwinkel sein, wie teilweise in der EP-0'924'513 A1 und insbesondere in den Absätzen [0022]-[0034] daraus beschrieben. Durch den Hinweis auf die EP-0'924'513 A1 erübrigt sich hier eine detaillierte Beschreibung der Methoden, die zum Zwischenkennwert 111 führen. Anschliessend wird in der vorliegenden Erfindung der Zwischenkennwert 111 mit der dritten Grösse 103 verknüpft. Dadurch entsteht ein Fremdstoffsignal 120, das von der Masse der Prüfguts und der Feuchtigkeit im Prüfgut im Wesentlichen unabhängig ist.

Eine weitere Ausführungsform des erfindungsgemässen Verfahrens zeigt **Figur 2(c)****.** Auch hier werden eine erste Grösse 101 und eine zweite Grösse 102 bei ein und derselben Frequenz f1 aufgenommen, während eine dritte Grösse 103 bei einer anderen Frequenz f2 ≠ f1 aufgenommen wird. Die zweite Grösse 102 und die dritte Grösse 103 werden zu einem von der Masse des Prüfgut unabhängigen Zwischenkennwert 112 verknüpft. Der Zwischenkennwert 112 kann z. B. eine Dielektrizitätszahl, eine Dielektrizitätskonstante (Permittivität) oder eine Kapazität sein. Anschliessend wird der Zwischenkennwert 112 mit der ersten Grösse 101 verknüpft, wodurch ein Fremdstoffsignal 120 entsteht, das von der Masse der Prüfguts und der Feuchtigkeit im Prüfgut im Wesentlichen unabhängig ist.

In der Ausführungsform von **Figur 2(d)** werden eine erste Grösse 101 sowie eine zweite Grösse 102 bei ein und derselben Frequenz f1, z. B. f1 = 10 MHz, aufgenommen, und eine dritte Grösse 103 sowie eine vierte Grösse 104 werden bei ein und derselben, anderen , Frequenz f2 ≠ f1, z. B. f2 = 200 kHz, aufgenommen. Die erste Grösse 101 und die zweite Grösse 102 werden zu einem ersten Zwischenkennwert 111, die dritte Grösse 103 und die vierte Grösse 104 zu einem zweiten Zwischenkennwert 113 verknüpft. Anschliessend werden der erste Zwischenkennwert 111 und der zweite Zwischenkennwert 113 zu einem Fremdstoffsignal 120 verknüpft, das von der Masse der Prüfguts und der Feuchtigkeit im Prüfgut im Wesentlichen unabhängig ist.

Wie ein Vergleich der Figuren 2 und 1 zeigt, unterscheiden sich das erfindungsgemässe Verfahren insbesondere durch folgende Merkmale vom aus der EP-0'924'513 A1 bekannten Verfahren:
- Es werden mindestens drei elektrische Grössen 101-104 aufgenommen.
- Die Aufnahme der elektrischen Grössen 101-104 erfolgt bei mindestens zwei verschiedenen Frequenzen f1-f3.
- Es wird kein Vergleich mit einem für ein bestimmtes Fremdstoffmaterial charakteristischen Referenzwert durchgeführt.
- Das Ausgangssignal 120 ist nicht nur von der Masse der Prüfguts, sondern auch von der Feuchtigkeit im Prüfgut unabhängig.

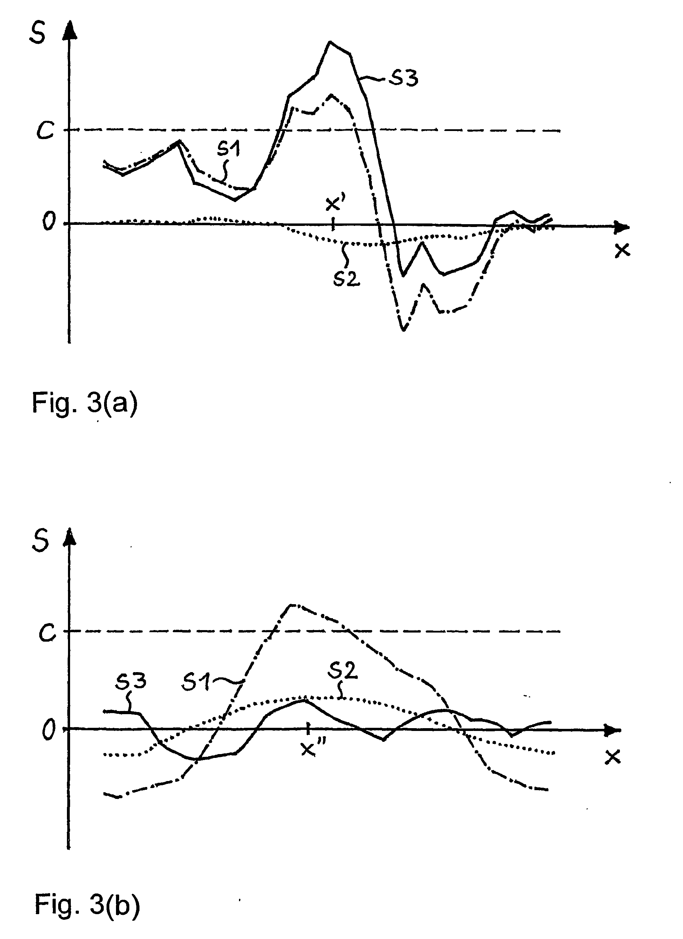

In den Diagrammen von **Figur 3** sind beispielhafte Messsignale S gegenüber einer Position x auf dem Garn aufgetragen. Die Signale S könnten der Ausführungsform von Fig. 2(b) entstammen. Eine erste Kurve S1 zeigt den Zwischenkennwert 111, eine zweite Kurve S2 die dritte Messgrösse 103, und eine dritte Kurve S3 das Fremdstoffsignal 120, das eine Linearkombination des Zwischenkennwerts S 1 und der dritten Messgrösse sein kann, z. B. S3 = S1 - 3·S2.

**Figur 3(a)** zeigt den Fall, in dem sich im Bereich einer Garnstelle x' ein zu erkennender fester Fremdstoffpartikel, z. B. ein Polypropylenstreifen, befindet. In diesem Bereich steigt der Zwischenkennwert S1 an, während die dritte Messgrösse S2 auf leicht negative Werte absinkt. Das Fremdstoffsignal S3 steigt dementsprechend noch stärker an und zeigt beim Fremdstoffpartikel eine ausgeprägte Erhebung. Bei einem Garnreiniger wird üblicherweise eine obere Reinigungsgrenze C vordefiniert. Wenn das Fremdstoff- oder Reinigersignal S3 die Reinigungsgrenze C übersteigt, wird ein Garnschnitt ausgelöst. Dies würde sowohl beim Zwischenkennwert S1 als auch - noch ausgeprägter - beim Fremdstoffsignal S3 zutreffen.

**Figur 3(b)** zeigt den Fall, in dem sich im Bereich einer Garnstelle x" eine zu übergehende lokale Feuchtestelle befindet. In diesem Bereich steigt der Zwischenkennwert S1 ebenso wie beim Fremdstoffpartikel von Fig. 3(a) an und übersteigt die Reinigungsgrenze C. Die dritte Messgrösse S2 ist aber auch feuchteempfindlich und steigt ebenfalls an, so dass das Fremdstoffsignal S3 um Null herum bleibt und die Reinigungsgrenze C nicht übersteigt.

Im Beispiel von Fig. 3(a) wäre zu Recht schon dann ein Garnschnitt ausgelöst worden, wenn - wie in der EP-0'924'513 A1 beschrieben - nur der Zwischenkennwert S 1 als Reinigersignal verwendet worden wäre. Die Linearkombination S3 steigt beim Fremdstoffpartikel noch stärker an und löst somit- ebenfalls zu Recht - auch einen Garnschnitt aus. Der eigentliche Vorteil der Erfindung wird erst anhand von Fig. 3(b) ersichtlich. Dort würde der Zwischenkennwert S 1 einen Fehlschnitt auslösen. Erst die erfindungsgemässe Verknüpfung des Zwischenkennwerts S1 mit der dritten Messgrösse S2 zu einem Fremdstoffsignal S3 vermag zwischen festen Fremdstoffpartikeln und lokalen Feuchtigkeitsschwankungen zu unterscheiden. Dank der Erfindung werden also Fehlschnitte, wie sie das Signal S1 in Fig. 3(b) auslösen würde, vermieden.

In **Figur 4(a)** ist ein elektrisches Schaltschema einer ersten Ausführungsform der erfindungsgemässen Vorrichtung 1 dargestellt. Diese Ausführungsform führt das Auswerteverfahren gemäss Fig. 2(b) aus. Ein Messkondensator 2 weist zwei parallele Kondensatorplatten 21, 22 und eine dazwischen liegende Durchgangsöffnung 20 für ein entlang seiner Längsachse bewegtes, festes, längliches Prüfgut 9 wie Garn auf. Fakultativ ist in der Vorrichtung 1 ein Referenzkondensator 3 zur Ausschaltung oder Verringerung von unerwünschten Umgebungseinflüssen wie Luftfeuchtigkeit oder Lufttemperatur vorhanden. Der Referenzkondensator 3 ist vorzugsweise gleich aufgebaut wie der Messkondensator 2 und parallel zu diesem geschaltet, mit dem Unterschied, dass er nicht vom Prüfgut 9 durchlaufen wird. Die Vorrichtung 1 beinhaltet ferner einen ersten Teilwechselspannungsgenerator 41 zum Erzeugen einer ersten Wechselspannungskomponente mit einer ersten Frequenz, z. B. aus dem Bereich zwischen 1 MHz und 100 MHz, vorzugsweise zwischen 5 MHz und 50 MHz und bspw. ungefähr gleich 10 MHz. Die Vorrichtung 1 beinhaltet auch einen zweiten Teilwechselspannungsgenerator 42 zum Erzeugen einer zweiten Wechselspannungskomponente mit einer zweiten Frequenz, z. B. aus dem Bereich zwischen 10 kHz und 1000 kHz, vorzugsweise zwischen 50 kHz und 500 kHz und bspw. ungefähr gleich 200 kHz. Die erste und die zweite Wechselspannungskomponente sind vorzugsweise sinusförmig. Sie werden einander in einem analogen Spannungsaddierer 43 mit differenziellem Ausgang überlagert. Der erste und zweite Teilwechselspannungsgenerator 41, 42 sowie der Spannungsaddierer 43 können, zumindest gedanklich, zu einem Spannungsgenerator 4 zusammengefasst werden, der eine resultierende, aus zwei Wechselspannungskomponenten zusammengesetzte Wechselspannung erzeugt. Die resultierende Wechselspannung wird am Messkondensator 2 und am Referenzkondensator 3 angelegt.

Selbstverständlich können auch andere, an sich bekannte Arten von Spannungsgeneratoren 4 für die erfindungsgemässe Vorrichtung 1 verwendet werden. So ist es nicht unbedingt nötig, zwei sinusförmige Wechselspannungskomponenten mit zwei Teilwechselspannungsgeneratoren 41, 42 zu erzeugen. Alternativ kann z. B. eine Wechselspannung in Form von periodischen Rechteckpulsen erzeugt werden, deren Fourierentwicklung bekanntlich sinusförmige Komponenten mit Frequenzen enthält, die ein ungeradzahliges Vielfaches der Grundfrequenz sind. Beliebige dieser Komponenten, inklusive der Grundfrequenz, können zur Messung verwendet werden.

Den Kondensatoren 2, 3 ist vorzugsweise eine Detektorschaltung 5 zur Detektion eines auf einer elektrischen Leitung 51 ankommenden analogen Ausgangssignals der Kondensatoren 2, 3 nachgeschaltet. Im Ausführungsbeispiel von Fig. 4(a) beinhaltet die Detektorschaltung 5 zunächst einen Verstärker 52 zur Verstärkung des Ausgangssignals. Das Ausgangssignal des Verstärkers 52 wird einer Frequenzweiche 53 zugeführt, welche das Ausgangssignal in eine erste und eine zweite Komponente, entsprechend den beiden angelegten Frequenzen, aufteilt und diese Komponenten auf einen ersten Signalpfad 6 bzw. einen zweiten Signalpfad 7 führt. Dies kann z. B. mit einem Hochpassfilter 54 und einem Tiefpassfilter 55 bewerkstelligt werden. Auf dem ersten, hochfrequenten Signalpfad 6 wird die erste Signalkomponente nochmals auf zwei Teilpfade 61, 62 aufgeteilt und bei zwei verschiedenen Phasen demoduliert. Die Demodulation wird im Wesentlichen synchron, als eine Multiplikation der Teilsignalkomponenten mit dem an den Kondensatoren 2, 3 angelegten ersten Wechselspannungssignal mittels Multiplizierer 63, 64 ausgeführt. Die Phasenverschiebung wird mit einem Phasenschieber 66, der vorzugsweise als RC-Phasenschieber ausgebildet ist, erreicht. Sie beträgt vorzugsweise 90°, um ein Quadratursignal zu erhalten. Es ist aber durchaus möglich, eine andere Phasenschiebung zu wählen. Auf dem zweiten, tieffrequenten Signalpfad 7 wird die zweite Signalkomponente auf gleiche Weise mit Hilfe eines Multiplizierers 73 demoduliert. Zur Glättung werden alle drei Signale durch je einen Tiefpassfilter 56-58 geschickt und danach einer Auswerteeinheit 8 zugeführt.

Die Auswerteeinheit 8 kann eine analoge elektrische Schaltung oder eine digitale Schaltung mit einem Prozessor beinhalten. Im Ausführungsbeispiel von Fig. 4(a) umfasst sie ein Zwischenauswertemodul 81 zur Verknüpfung der beiden Teilsignalkomponenten des ersten, hochfrequenten Signalpfads 6 zu einem Zwischenkennwert. Der Zwischenkennwert wird danach in der Auswerteeinheit 8 mit der zweiten Signalkomponente zu einem resultierenden Fremdstoffsignal verknüpft, das die Erkennung von Fremdstoffen im Prüfgut 9 erlaubt. Ein Beispiel für ein derartiges Fremdstoffsignal ist die Linearkombination S3 von Fig. 3. Mit geeigneten Auswertemethoden ist es sogar möglich, auch den quantitativen Anteil der Fremdstoffe und allenfalls das Material der Fremdstoffe zu bestimmen. Verfahren und Vorrichtungen zur Quantifizierung von festen Fremdstoffen in textilem Prüfgut sind aus der EP-0'924'513 A1 bekannt und können auch für die vorliegende Erfindung übernommen werden. Das Fremdstoffsignal kann als Ausgangssignal der Vorrichtung auf einer Ausgangsleitung 89 ausgegeben werden. Alternativ dazu kann das Fremsdstoffsignal in der Auswerteeinheit 8 weiter verarbeitet, bspw. mit einer vorgegebenen Reinigungsgrenze C (vgl. Fig. 3) verglichen werden. Diesfalls besteht ein mögliches Ausgangssignal in einem Befehl "schneiden" oder "nicht schneiden", der an eine Schneidvorrichtung eines Garnreinigers erteilt wird.

**Figur 4(b)** zeigt ein elektrisches Schaltschema einer zweiten Ausführungsform der erfindungsgemässen Vorrichtung 1. Der Unterschied zur ersten Ausführungsform von Fig. 4(a) besteht darin, dass die zweite Signalkomponente auf dem zweiten, tieffrequenten Signalpfad 7 ebenfalls auf zwei Teilpfade 71, 72 aufgeteilt und bei zwei verschiedenen Phasen demoduliert wird. Dies entspricht dem Auswerteverfahren gemäss Fig. 2(d). Der zweite Signalpfad beinhaltet, analog zum ersten Signalpfad 6, zwei Multiplizierer 73, 74 und einen Phasenschieber 76. Die Auswerteeinheit 8 umfasst ein erstes Zwischenauswertemodul 81 zur Verknüpfung der beiden Teilsignalkomponenten des ersten, hochfrequenten Signalpfads 6 zu einem ersten Zwischenkennwert und ein zweites Zwischenauswertemodul 82 zur Verknüpfung der beiden Teilsignalkomponenten des zweiten, tieffrequenten Signalpfads 7 zu einem zweiten Zwischenkennwert. Der erste und der zweite Zwischenkennwert werden in der Auswerteeinheit 8 zu einem Ausgangssignal verknüpft. Die übrigen Elemente der Schaltung von Fig. 4(b) sind dieselben und tragen dieselben Bezugszeichen wie in der ersten Ausführungsform von Fig. 4(a).

Selbstverständlich ist die vorliegende Erfindung nicht auf die oben diskutierten Ausführungsformen beschränkt. So können z. B. mehr als vier Grössen bei mehr als zwei verschiedenen Frequenzen gemessen und miteinander verknüpft werden. Es sei hier nochmals hervorgehoben, dass die oben diskutierten Beispiele, die sich auf die Garnreinigung beziehen, die Allgemeinheit der Erfindung nicht einschränken. Weitere mögliche Anwendungen liegen z. B. auf dem Gebiet der Laborprüfung von Garnen. Dort werden hauptsächlich die Garnmasse und die Garnfeuchtigkeit interessieren, die dank der vorliegenden Erfindung unabhängig voneinander ermittelt werden können. Bei Kenntnis der Erfindung wird der Fachmann weitere Varianten herleiten können, die auch zum Gegenstand der vorliegenden Erfindung gehören.

### BEZUGSZEICHENLISTE

- 1: Vorrichtung

- 2: Messkondensator
- 20: Durchgangsöffnung
- 21, 22: Kondensatorplatten

- 3: Referenzkondensator

- 41, 42: erster, zweiter Wechselspannungsgenerator
- 43: Spannungsaddierer

- 5: Detektorschaltung
- 51: Leitung
- 52: Verstärker
- 53: Frequenzweiche
- 54: Hochpassfilter
- 55: Tiefpassfilter
- 56-59: Tiefpassfilter

- 6: erster Signalpfad
- 61,62: erster, zweiter Teilpfad
- 63, 64: Multiplizierer
- 66: Phasenschieber

- 7: zweiter Signalpfad
- 71, 72: erster, zweiter Teilpfad
- 73, 74: Multiplizierer
- 76: Phasenschieber

- 8: Auswerteeinheit
- 81, 82: erstes, zweites Zwischenauswertemodul
- 89: Ausgangsleitung

- 9: Prüfgut

- 101-104: Messgrössen
- 111-113: Zwischenkennwerte
- 120: Fremdstoffsignal

- 201, 202: Messgrössen
- 211: Kennwert
- 220: Anteil des Fremdstoffs
- 231: Referenzwert

- C: Reinigungsgrenze
- S: Signal
- S1: Signal des Zwischenkennwertes
- S2: Signal der dritten Messgrösse
- S3: Fremdstoffsignal
Position auf Garn
- x x': Garnstelle mit Fremdstoffpartikel
- x": Feuchtestelle

## Patentansprüche

1. Verfahren zur Untersuchung von bewegtem, festem, länglichem Prüfgut (9), wobei ein elektrisches Wechselfeld erzeugt wird,
welches eine Überlagerung mindestens einer ersten Wechselfeldkomponente mit einer ersten Frequenz (f1) und einer zweiten Wechselfeldkomponente mit einer von der ersten Frequenz (f1) verschiedenen zweiten Frequenz (f2) ist, das Prüfgut (9) dem Wechselfeld ausgesetzt wird,
Eigenschaften des mit dem Prüfgut (9) wechselwirkenden Wechselfeldes ermittelt werden, indem
eine vom Wechselfeld beeinflusste elektrische Grösse (101) bei der ersten Frequenz (f1) aufgenommen wird,
eine andere vom Wechselfeld beeinflusste elektrische Grösse (102) bei der zweiten Frequenz (f2) aufgenommen wird und
die aufgenommenen elektrischen Grössen (101, 102) miteinander verknüpft werden,
wobei
mindestens drei vom Wechselfeld beeinflusste elektrische Grössen (101-104) bei jeweils einer Frequenz (f1-f3) aufgenommen werden,
wovon eine (101) der Grössen (101-104) bei der ersten Frequenz (f1) und eine andere (102) der Grössen (101-104) bei der zweiten Frequenz (f2) aufgenommen wird, und
die mindestens drei Grössen (101-104) miteinander verknüpft werden.

2. Verfahren nach Anspruch 1, wobei die Verknüpfung die Bildung einer Linearkombination beinhaltet.

3. Verfahren nach Anspruch 1 oder 2, wobei zwei Grössen (101, 102) bei der ersten Frequenz (f1) aufgenommen und miteinander zu einem Zwischenkennwert (111) verknüpft werden und anschliessend eine dritte Grösse (103) bei der zweiten Frequenz (f2) aufgenommen und mit dem Zwischenkennwert (111) verknüpft wird.

4. Verfahren nach Anspruch 3, wobei der Zwischenkennwert (111) eine Phasenverschiebung, ein Leistungsfaktor oder ein Verlustwinkel ist.

5. Verfahren nach Anspruch 1 oder 2, wobei zwei Grössen (102, 103) bei der ersten Frequenz (f1) bzw. bei der zweiten Frequenz (f2) aufgenommen und miteinander zu einem Zwischenkennwert (112) verknüpft werden und anschliessend eine dritte Grösse (101) mit dem Zwischenkennwert (112) verknüpft wird.

6. Verfahren nach Anspruch 5, wobei der Zwischenkennwert (112) eine Dielektrizitätszahl, eine Dielektrizitätskonstante oder eine Kapazität ist.

7. Verfahren nach einem der vorangehenden Ansprüche, wobei die erste Frequenz (f1) zwischen 1 MHz und 100 MHz, vorzugsweise zwischen 5 MHz und 50 MHz und bspw. bei ca. 10 MHz liegt, und die zweite Frequenz (f2) zwischen 10 kHz und 1000 kHz, vorzugsweise zwischen 50 kHz und 500 kHz und bspw. bei ca. 200 kHz liegt.

8. Verfahren nach einem der vorangehenden Ansprüche, wobei aus der Verknüpfung der mindestens drei Grössen (101-104) ein Fremdstoffsignal, ein Massensignal und/oder ein Feuchtesignal ermittelt wird.

9. Vorrichtung (1) zur Untersuchung von bewegtem, festem, länglichem Prüfgut (9), beinhaltend
einen Messkondensator (2) mit einer Durchgangsöffnung (20) für das Prüfgut (9), einen Wechselspannungsgenerator (4) zum Anlegen einer Wechselspannung an den Messkondensator (2) zwecks Beaufschlagung der Durchgangsöffnung (20) mit einem elektrischen Wechselfeld,
welcher Wechselspannungsgenerator (4) geeignet ist, die Durchgangsöffnung (20) mit einem Wechselfeld zu beaufschlagen, das eine Überlagerung mindestens einer ersten Wechselfeldkomponente mit einer ersten Frequenz (f1) und einer zweiten Wechselfeldkomponente mit einer von der ersten Frequenz (f1) verschiedenen zweiten Frequenz (f2) ist, und
eine mit dem Messkondensator (2) elektrisch verbundene Auswerteschaltung (8) zur Ermittlung von Eigenschaften des mit dem Prüfgut (9) wechselwirkenden Wechselfeldes,
welche Auswerteschaltung (8) geeignet ist, eine vom Wechselfeld beeinflusste, bei der ersten Frequenz (f1) aufgenommene elektrische Grösse (101) und eine andere vom Wechselfeld beeinflusste, bei der zweiten Frequenz (f2) aufgenommene elektrische Grösse miteinander zu verknüpfen,
wobei
die Auswerteschaltung (8) geeignet ist, mindestens drei vom Wechselfeld beeinflusste, bei jeweils einer Frequenz (fl-f3) aufgenommene elektrische Grössen (101-104) miteinander zu verknüpfen,
wovon eine (101) der Grössen (101-104) bei der ersten Frequenz (f1) und eine andere (102) der Grössen (101-104) bei der zweiten Frequenz (f2) aufgenommen ist.

10. Vorrichtung (1) nach Anspruch 9, wobei zwischen dem Messkondensator (2) und der Auswerteschaltung (8) eine Detektorschaltung (5) zur Detektion eines Ausgangssignals des Messkondensators (2) angeordnet ist.

11. Vorrichtung (1) nach Anspruch 10, wobei die Detektorschaltung (5) eine Frequenzweiche (53) zur Aufteilung des Ausgangssignals auf zwei Signalpfade (6, 7), von denen ein erster Signalpfad (6) der Übertragung eines Ausgangsteilsignals der ersten Frequenz (f1) und ein zweiter Signalpfad (7) der Übertragung eines Ausgangsteilsignals der zweiten Frequenz (f2) dient, beinhaltet.

12. Vorrichtung (1) nach einem der Ansprüche 9-11, wobei die Vorrichtung (1) nebst dem Messkondensator (2) einen Referenzkondensator (3) beinhaltet, der vorzugsweise zum Messkondensator (2) parallel geschaltet ist.

13. Verwendung der Vorrichtung (1) nach einem der Ansprüche 9-12 zur Erkennung eines festen Fremdstoffs, zur Ermittlung einer Masse pro Längeneinheit und/oder zur Ermittlung einer Feuchte.

## Claims

1. A method for examining a moved, solid, elongate product to be tested (9), wherein an electrical alternating field is produced,
which alternating field is a superposition of at least one first alternating field component with a first frequency (f1), and of a second alternating field component with a second frequency (f2) which is different from the first frequency (f1),
the product to be tested (9) is subjected to the alternating field,
characteristics of the alternating field interacting with the product to be tested (9) are evaluated in that
an electrical variable (101) influenced by the alternating field is recorded at the first frequency (f1),
another electrical variable (102) influenced by the alternating field is recorded at the second frequency (f2), and
the recorded electrical variables (101, 102) are recorded and combined with one another,
wherein
at least three electrical variables (101-104) influenced by the alternating field are recorded in each case at one frequency (fl-f3),
of which one (101) of the variables (101-104) is recorded at the first frequency (f1), and another (102) of the variables (101-104) is recorded at the second frequency (f2), and the at least three variables (101-104) are combined with one another.

2. The method according to claim 1, wherein the combination includes the formation of a linear combination.

3. The method according to claim 1 or 2, wherein two variables (101, 102) are recorded at the first frequency (f1) and combined with one another into an intermediate characteristic value (11), and subsequently a third variable (103) is recorded at a second frequency (f2) and combined with the intermediate characteristic value (111).

4. The method according to claim 3, wherein the intermediate characteristic value (111) is a phase shift, a power factor or a loss angle.

5. The method according to claim 1 or 2, wherein two variables (102, 103) are recorded at the first frequency (f1) and at the second frequency (f2) respectively, and combined with one another into an intermediate characteristic value (112), and subsequently a third variable (101) is combined with the intermediate characteristic value (112).

6. The method according to claim 5, wherein the intermediate characteristic value (112) is a dielectric constant, a relative permittivity or a capacitance.

7. The method according to one of the preceding claims, wherein the first frequency (f1) lies between 1 MHz and 100 MHz, preferably between 5 MHz and 50 MHz, and for example at approx. 10 MHz, and the second frequency (f2) lies between 10 kHz and 1000 kHz, preferably between 50 kHz and 500 kHz and for example at approx. 200 kHz.

8. The method according to one of the preceding claims, wherein a foreign matter signal, a mass signal and/or a humidity signal is evaluated from the combination of the at least three variables (101-104).

9. A device (1) for examining a moved, solid, elongate product to be tested (9), containing a measurement capacitor (2) with a through-opening (20) for the product to be tested (9),
an alternating voltage generator (4) for applying an alternating voltage to the measurement capacitor (2) for the purpose of subjecting the through-opening (20) to an electrical alternating field,
which alternating voltage generator (4) is suitable for subjecting the through-opening (20) to an alternating field, which is a superposition of at least a first alternating field component with a first frequency (f1), and of a second alternating field component with a second frequency (f2) different from the first frequency (f1), and
an evaluation circuit (8) electrically connected to the measurement capacitor (2), for determining properties of the alternating field interacting with the product to be tested (9),
which evaluation circuit (8) is suitable for combining a first electrical variable (101), which is influenced by the alternating field and is recorded at the first frequency (f1), and another electrical variable, which is influenced by the alternating field and is recorded at the second frequency (f2), with one another,
wherein
the evaluation circuit (8) is suitable for combining at least three electrical variables (101-104) with one another, which are influenced by the alternating field and are recorded in each case at one frequency (f1-f3),
of which one (101) of the variables (101-104) is recorded at the first frequency (f1) and another (102) of the variables (101-104) is recorded at the second frequency (f2).

10. The device (1) according to claim 9, wherein a detector circuit (5) for detecting an output signal of the measurement capacitor (2) is arranged between the measurement capacitor (2) and the evaluation circuit (8).

11. The device (1) according to claim 10, wherein the detector circuit (5) contains a diplexer (53) for dividing the output signal onto two signal paths (6, 7), of which a first signal path (6) serves for transmitting an output part signal of the first frequency (f1), and a second signal path (7) serves for transmitting an output part signal of the second frequency (f2).

12. The device (1) according to one of the claims 9-11, wherein the device (1) apart from the measurement capacitor (2) contains a reference capacitor (3) which is preferably connected in parallel to the measurement capacitor (2).

13. A use of a device (1) according to one of the claims 9-12 for recognizing solid foreign matter, for evaluating a mass per length unit and/or for determining a humidity.

## Revendications

1. Procédé pour l'examen d'objet à examiner (9) solide et allongé en mouvement, dans lequel
un champ électrique alternatif est produit,
qui est une superposition d'au moins une première composante de champ alternatif à une première fréquence (f1) et d'une deuxième composante de champ alternatif à une deuxième fréquence (f2) différente de la première fréquence (f1),
l'objet à examiner (9) est exposé au champ alternatif,
des propriétés du champ alternatif interagissant avec l'objets à examiner (9) sont déterminées
en acquérant une grandeur électrique (101) influencée par le champ alternatif à la première fréquence (f1),
en acquérant une autre grandeur électrique (102) influencée par le champ alternatif à la deuxième fréquence (f2) et
en associant entre elles les grandeurs électriques (101, 102) acquises,
dans lequel
au moins trois grandeurs électriques (101-104) influencées par le champ alternatif sont acquises chacune à une fréquence (fl-f3),
l'une (101) des grandeurs (101-104) étant acquise à la première fréquence (f1) et une autre (102) des grandeurs (101-104) étant acquise à la deuxième fréquence (f2), et les au moins trois grandeurs (101-104) sont associées entre elles.

2. Procédé selon la revendication 1, dans lequel l'association comprend la formation d'une combinaison linéaire.

3. Procédé selon la revendication 1 ou 2, dans lequel deux grandeurs (101, 102) sont acquises à la première fréquence (f1) et associées l'une à l'autre pour donner une valeur caractéristique intermédiaire (111), puis une troisième grandeur (103) est acquise à la deuxième fréquence (f2) et associée à la valeur caractéristique intermédiaire (111).

4. Procédé selon la revendication 3, dans lequel la valeur caractéristique intermédiaire (111) est un décalage de phase, un facteur de puissance ou un angle de perte.

5. Procédé selon la revendication 1 ou 2, dans lequel deux grandeurs (102, 103) sont acquises à la première fréquence (f1) ou à la deuxième fréquence (f2) et associées l'une à l'autre pour donner une valeur caractéristique intermédiaire (112), puis une troisième grandeur (101) est associée à la valeur caractéristique intermédiaire (112).

6. Procédé selon la revendication 5, dans lequel la valeur caractéristique intermédiaire (112) est un facteur diélectrique, une constante diélectrique ou une capacité.

7. Procédé selon l'une des revendications précédentes, dans lequel la première fréquence (f1) est comprise entre 1 MHz et 100 MHz, de préférence entre 5 MHz et 50 MHz, et se situe par exemple à environ 10 MHz, et la deuxième fréquence (f2) est comprise entre 10 kHz et 1000 kHz, de préférence entre 50 kHz et 500 kHz, et se situe par exemple à environ 200 kHz.

8. Procédé selon l'une des revendications précédentes, dans lequel un signal de matière étrangère, un signal de masse et/ou un signal d'humidité est déterminé à partir de l'association des au moins trois grandeurs (101-104).

9. Dispositif (1) pour l'examen d'objet à examiner (9) solide et allongé en mouvement, comprenant
un condensateur de mesure (2) avec une ouverture de passage (20) pour l'objet à examiner (9),
un générateur de courant alternatif (4) pour appliquer un courant alternatif au condensateur de mesure (2) afin d'exposer l'ouverture de passage (20) à un champ électrique alternatif,
lequel générateur de courant alternatif (4) convient pour exposer l'ouverture de passage (20) à un champ alternatif qui est une superposition d'au moins une première composante de champ alternatif à une première fréquence (f1) et d'une deuxième composante de champ alternatif à une deuxième fréquence (f2) différente de la première fréquence (f1), et
un circuit d'analyse (8) connecté électriquement au condensateur de mesure (2) pour déterminer les propriétés du champ alternatif interagissant avec l'objet à examiner (9),
lequel circuit d'analyse (8) convient pour associer entre elles une grandeur électrique (101) influencée par le champ alternatif acquise à la première fréquence (f1) et une autre grandeur électrique influencée par le champ alternatif acquise à la deuxième fréquence (f2),
dans lequel
le circuit d'analyse (8) convient pour associer entre elles au moins trois grandeurs électriques (101-104) influencées par le champ alternatif acquises chacune à une fréquence (f1-f3),
l'une (101) des grandeurs (101-104) étant acquise à la première fréquence (f1) et une autre (102) des grandeurs (101-104) étant acquise à la deuxième fréquence (f2).

10. Dispositif (1) selon la revendication 9, dans lequel est prévu entre le condensateur de mesure (2) et le circuit d'analyse (8) un circuit de détecteur (5) pour la détection d'un signal de sortie du condensateur de mesure (2).

11. Dispositif (1) selon la revendication 10, dans lequel le circuit de détecteur (5) présente un diviseur de fréquences (53) pour diviser le signal de sortie sur deux voies de signal (6, 7), la première voie de signal (6) servant à la transmission d'un signal de sortie partiel à la première fréquence (f1) et une deuxième voie de signal (7) à la transmission d'un signal de sortie partiel à la deuxième fréquence (f2).

12. Dispositif (1) selon l'une des revendications 9 à 11, dans lequel le dispositif (1) contient outre le condensateur de mesure (2) un condensateur de référence (3) qui est de préférence monté en parallèle avec le condensateur de mesure (2).

13. Utilisation du dispositif (1) selon l'une des revendications 9 à 12 pour la détection d'une matière étrangère, pour la détermination d'une masse par unité de longueur et/ou pour la détermination d'une humidité.
